# EUROPEAN PATENT APPLICATION

(11) **EP 2 020 224 A1**
(43) Date of publication of application: **04.02.2009**
(21) Application number: 08104866.2
(22) Date of filing: 24.07.2008
(51) Int. Cl.: A61K 8/11, A61K 8/25, A61K 8/44, A61K 8/891, A61Q 19/00

(54) **Compositions containing betaine and hydrophobic silica**

(30) Priority: 30.07.2007 EP 07113456
(71) Applicant: Sorex Limited, Widnes, Cheshire WA8 8TJ (GB)
(72) Inventor: Twydell, Roland, Cheshire WA8 8TJ (GB)
(74) Representative: Wilkinson, Stephen John

(57) **Abstract**

A free-flowing, substantially dry-to-the-touch, particulate composition comprises (A) finely particulate hydrophobic silicone-treated silica having a surface area of from 80 to 300 m²/g, (B) trimethylglycine, and (C) water, wherein the content of component (A) is greater than 2% and less than 8% by weight based on the total weight of components (A), (B) and (C) and wherein the content of component (B) is greater than 30% and less than 65% by weight based on the total weight of components (A), (B) and (C), said composition being in the form of particles of an aqueous solution of component (B) the surfaces of which particles are coated with a coating of component (A).

The particulate composition is useful as a moisturising component in skin-care or cosmetic formulations.

## Description

The present invention relates to compositions containing betaine and hydrophobic silica. It, further, relates to the use of such compositions as moisturising components in skin-care and cosmetic formulations.

Aqueous dispersions of silica can be prepared into a state known generally in the prior art as "dry water". "Dry water" is known in two forms. The first can be produced by absorbing aqueous liquids onto hydrophilic material to form a substance which exists as free-flowing powder or granules. The second form can be produced by coating finely divided aqueous liquids with powdered hydrophobic material, such as metal oxides. Each liquid particle, in this second form of dry water, is separated from the next by hydrophobic metal oxide coatings and by air spaces. In order to create this second form of dry water, it is necessary to subject a mixture of the aqueous liquid and hydrophobic metal oxide to high shear mixing conditions using a high speed mixer at mixing speeds typically in excess of 6000 rpm for over 15 minutes. Unfortunately, this second form of dry water is thermodynamically unstable and, when produced, tends to break down after a relatively short period of time.

A dry water composition containing pyrogenically-produced hydrophobic silica is disclosed in US 5,122,518. This composition is described as being useful for controlling insects and other pests. The dry water composition prepared according to this prior art document is, however, unstable and cannot be stored for long periods of time.

A composition comprising a storage-stable aerated gel is disclosed in WO 01/35744. This composition is in the form of fine particles of an aqueous gel containing water and a gelling agent selected from xanthan gum, sodium alginate and neutralised carboxyvinyl polymer wherein the surfaces of the fine particles of aqueous gel are covered with a coating of finely particulate hydrophobic silica. The composition disclosed in this prior art document is a free-flowing pulverulent fluid composition.

Particulate compositions in the form of fine particles of an aqueous solution of a water-soluble saccharide wherein the surfaces of the particles of the aqueous solution are coated with a coating of finely particulate amorphous hydrophobic silica are disclosed in WO 03/045152. The disclosed particulate compositions are biocidally-active against insects, mites and lice. Such compositions are substantially dry to the touch until direct pressure is exerted whereupon the substantially dry-to-the-touch particulate composition becomes transformed into a sticky cream-like or paste-like material.

The prior art documents WO 01/35744 and WO 03/045152, referred to above, show that certain specified gelling agents and saccharides have the ability to stabilise dry water compositions such that water-containing, yet substantially dry-to-the-touch, particulate compositions can be manufactured which are stable under storage.

Moisturising compositions are used in cosmetic and skin-care formulations. True moisturisers are substances which determine the water balance of the skin on a cellular level. They have the ability to influence and/or control the water balance in the cells and the extra-cellular fluid. The term "moisturiser", however, is also commonly used to describe substances such as humectants which, because of their hygroscopicity, are able to bind water and emollients which make the outermost layer of the epidermis (stratum corneum) more soft and supple. In addition to imparting or restoring normal levels of hydration to the skin, moisturising compositions may also provide a protective barrier in the external layers of the skin which physically blocks the surface to reduce transepidermal water loss.

Betaine (trimethylglycine) is a well-known osmo-protectant occurring naturally in sugar beet (*Beta vulgaris*) and in a variety of other plants. It is a significant raw material in the manufacture of personal care and cosmetic products. It is specifically known to be useful in skin-care formulations as a moisturiser because of its water binding and cell membrane protective qualities. Cosmetic and/or dermatological preparations containing betaines are, for instance, disclosed in US 2004/0258722A. This document discloses preparations which contain betaines in a concentration of 0.01 to 75% by weight (based on the total weight of the preparation) and polyols, for example glycerol, in a concentration of 5 to 50% by weight (based on the total weight of the preparation).

JP-A-2004-043428 discloses a powder cosmetic composition which contains water, hydrophobic silica, a polyhydric alcohol and 0.1 to 10% of one or more specified amino acids or their salts. The document explains that the addition of a small amount of a specified amino acid to the aqueous component of the cosmetic comprising water and polyhydric alcohol overcomes problems arising from the use of the aqueous component containing the polyhydric alcohol.

The present invention is based on the surprising discovery that betaine has the ability to stabilise dry water compositions such that a betaine-containing dry water composition which is stable under storage can be manufactured. We have discovered that such a betaine-containing dry water composition is substantially dry-to-the-touch but which, when rubbed into the surface of the skin, transforms to a relatively non-sticky cream-like consistency which is absorbed by, and which moisturises, the skin.

Accordingly, the present invention provides a particulate composition comprising the components
(A) finely particulate hydrophobic silicone-treated silica having a surface area of from 80 to 300 m²/g;
(B) trimethylglycine; and
(C) water,
wherein component (A) is present in the composition in an amount greater than 2% by weight but less than 8% by weight based on the total weight of components (A), (B) and (C), and wherein component (B) is present in the composition in an amount greater than 30% by weight but less than 65% by weight based on the total weight of components (A), (B) and (C), the said particulate composition being in the form of particles of an aqueous solution of component (B) wherein the surfaces of the particles of the aqueous solution are coated with a coating of component (A).

The particulate composition of the present invention contains water, betaine and finely particulate hydrophobic silica. It is not a simple mixture of these components but has a specific form. The specific form of the composition of the invention is that it exists as particles of an aqueous solution of the betaine. The surfaces of these particles of betaine solution are coated with fine particles of amorphous hydrophobic silica. Thus, a particle of the particulate composition of the invention has, as an outer particle wall, a covering comprising the hydrophobic silica powder, and has a liquid core, enrobed by or encapsulated within this silica powder covering, which core comprises an aqueous solution of the betaine. Thus, the hydrophobic silica powder coating on one particle prevents that particle from sticking to its neighbouring particles and, further, prevents the liquid core of the particle from coalescing or merging with the liquid cores of neighbouring particles. Thus, the particulate composition of the present invention is free-flowing and substantially dry-to-the-touch until the hydrophobic silica coatings on the particles are ruptured by the external application of force thereto.

Where the words "comprises" and "comprising" are used herein, it is to be understood that these have the meanings "includes" and "including", respectively, to the extent that the presence of one or more other materials is not excluded.

As stated above, the particulate composition contains a component (A) which is a finely particulate hydrophobic silicone-treated silica having a surface area in the range of from 80 to 300 m²/g. By the term "finely particulate", as applied to the hydrophobic silicone-treated silica, it is meant that this hydrophobic silica will have an average particle size of less than 100 µm, preferably less than 40 µm. The hydrophobic silicone-treated silica is a silica which has been rendered hydrophobic by surface treatment using one or more organosilicon compounds to produce, on the silicon dioxide surface, hydrophobic silicone groups. The technique of hydrophobicizing silica in this way is well known and such hydrophobic silicone treated silica is available commercially. We have found that good results are obtained using hydrophobic silicone-treated silica marketed under the name CAB-O-SIL ("CAB-O-SIL" is a trademark of Cabot Corporation). A particularly preferred hydrophobic silicone-treated silica for use in the present invention is CAB-O-SIL TS-720. However, other hydrophobic silicone-treated silicas may also be used in the present invention provided that they have a surface area within the range of from 80 to 300 m²/g and an average particle size of less than 100 µm and preferably less than 40 µm. The hydrophobic silica may also be one that has been surface treated to produce siloxane, as well as silicone groups attached to the silicon dioxide surface.

The hydrophobic silicone-treated silica is used, in the composition of the present invention, in an amount greater than 2% by weight but less than 8% by weight based on the combined weight of the silica, the betaine and the water in the composition. We have found that the use of a hydrophobic silicone-treated silica in an amount up to 2% by weight (based on the combined weight of the silica, betaine and water in the composition) does not enable the production of a free-flowing pulverulent product. We have, also, found that silica contents of 8% by weight and above provide particulate compositions which are excessively dusty, i.e. they contain excess hydrophobic silicone-treated silica which, in addition to creating a dust problem, is wasteful of a relatively expensive material. Preferably, the content of the hydrophobic silicone-treated silica in the compositions of the present invention will be in the range of from 3 to 5% by weight based on the combined weights of the hydrophobic silica, betaine and water in the composition since the use of such a content gives a product having good properties of being pulverulent and free-flowing without excess silica.

Component (B) in the particulate composition defined above is betaine, i.e. trimethylglycine. The betaine used in the present invention may be in the monohydrate form or in the anhydrous form. Betaine in both such forms are commercially available materials, for instance under the name "Betafin" from Danisco A/S. The betaine is used in the particulate composition of the invention in an amount greater than 30% by weight but less than 65% by weight, based on the combined weight of the hydrophobic silicone-treated silica, betaine and water in the particulate composition. Unfortunately, a product containing up to 30% by weight betaine has unacceptable physical and microbiological properties. For instance, products containing 30% by weight or less of betaine are not sufficiently resistant to microbe growth and do not impart adequate storage stability on the product. The upper limit of the betaine content is determined by the solubility limit for betaine in water. Thus, when betaine is used in an amount of 65% by weight, or above, there is an increased tendency for the betaine solution to contain undissolved betaine. We have found that a good compromise is achieved by the use of a betaine content in the range of 40 to 62% by weight, based on the combined weight of the betaine, water and hydrophobic silica in the particulate composition. Most preferably, the betaine is used in an amount of about 60% by weight on the basis given above.

It is to be noted that the presence of betaine in the aqueous phase of the particulate composition of the invention, in an amount greater than 30% by weight but less than 65% by weight, based on the total weight of the components (A), (B) and (C), enables the manufacture of a storage-stable composition having excellent properties without any requirement for the presence, in the aqueous phase, of a polyhydric alcohol, unlike in JP-A-2004-043428. According to a preferred embodiment of the invention, the aqueous solution of betaine contains no polyhydric alcohols.

The water used as component (C) in the particulate composition of the present invention may typically be tap water, although in cases where the particulate composition is intended for use as an additive in a dermatological formulation, it is preferred to use a purified grade of water.

The process for producing the particulate compositions of the present invention involves mixing an aqueous solution of betaine, obtained by dissolving the betaine (component (B)) in the water (component (C)), with the finely particulate hydrophobic silicone-treated silica under mixing conditions which create shear, i.e. mixing conditions which cause the aqueous solution of betaine to be finely fragmented into minute droplets. When such minute droplets of the betaine solution are created under the mixing conditions described above, they become dispersed within the finely particulate hydrophobic silicone-treated silica such that the surfaces of the minute droplets of betaine solution become coated with the silica particles and, thus, stabilised against coalescence with other minute droplets of solution to form larger droplets of solution. The expression "mixing conditions which create shear", as used above, will be well understood by the person skilled in the art of mixing or blending materials and whether or not a particular mixing apparatus is capable of mixing aqueous compositions under shear conditions will be known to one skilled in the art. Mixing conditions which create shear may be achieved by using standard high speed mixers, typically using mixing speeds in the range of 2000 to 6500 rpm for a few minutes, for example 2 to 5 minutes. Preferably, for compositions containing a betaine content towards the upper limit of the content range, i.e. greater than 60% by weight based on the combined weight of the betaine, hydrophobic silica and water in the composition, a mixer speed of about 2500 rpm is employed for about 2 minutes to achieve a particulate composition according to the invention. For compositions having a lower betaine content, e.g. less than 40% by weight, we prefer to use a higher shear for a slightly longer period of time, for instance, about 6300 rpm for about 5 minutes to produce the particulate composition of the invention.

The particulate composition of the invention, when subjected to external pressure, such as when pressed between the fingers or when rubbed onto the skin, transforms to a non-sticky cream which is easily absorbed into the skin and which has moisturising properties when absorbed into the skin. The particulate composition of the invention, thus, is useful as a moisturising component in skin-care or cosmetic formulations. Such formulations, typically, will contain the particulate composition of the present invention in an amount sufficient to provide a betaine content in the formulation in the range of from 0.05 to 50%, by weight, based on the weight of the formulation. Preferably, the particulate composition of the invention will be incorporated into a skin-care or cosmetic formulation in an amount to provide a betaine content in the formulation in the range of from 0.5 to 10%, and more preferably from 2 to 5%, by weight.

Such formulations also contain a dermatologically-acceptable and/or cosmetically-acceptable carrier. Amounts of the carrier may range broadly from 50 to 99.95% by weight of the formulation. Examples of acceptable carriers include one or more substances selected from water, emollients and humectants. The carrier will typically be aqueous, for instance in the form of a water-in-oil emulsion or an oil-in-water emulsion, as will be apparent to the person skilled in the art.

Emollients are generally used in formulations to provide medium to high spreading property as well as a softening effect on the stratum corneum. According to a preferred embodiment of the present invention, the emollient used will provide a high spreading value such as is conventionally required by body lotions and hand creams. Examples of emollients which are used in formulations to provide a high spreading value include fatty acid esters such as isopropyl stearate, isopropyl palmitate, isopropyl myristate and isostearyl isostearate.

The formulation may contain one or more other substances which have an emollient effect, such as lanosterol and Jojoba oil. Lanosterol is particularly preferred in formulations since it is able to produce a barrier in the skin to reduce transepidermal moisture loss.

If desired, the formulation may also contain one or more humectants, which are known to the person skilled in the art.

Other conventional ingredients in skin-care and/or cosmetic products include antioxidants, phospholipids, fragrance additives, vitamins and stabilizers.

A skin-care or cosmetic formulation may be prepared by mixing the desired amount of the particulate composition of the invention with the other components of the formulation. As is known to persons skilled in the art, it may be preferred to form a premix of two or more components of the formulation and then mix the premix with other ingredients in order to obtain greater homogeneity in the mixture or for maintaining the stability of one or more ingredients.

### EXAMPLES

### Example 1 (comparative)

To a 250ml glass beaker equipped with an IKA RE166 tooth-disc laboratory high-shear mixer were added 66.1g tap water, 30g betaine and 3.9g CAB-O-SIL TS-720 (a treated fumed silica from Cabot Corporation obtained by replacing many of the surface hydroxyl groups on the fumed silica with a polydimethyl-siloxane polymer to render the silica hydrophobic). CAB-O-SIL TS-720 has a B.E.T. surface area of 115 m²/g, a 325 Mesh Residue (44 microns) of 0.5% max., a specific gravity of 2.2 g/cm³ and refractive index of 1.46. The contents of the beaker, having an ambient temperature of 22°C, were mixed for 5 minutes at a mixing speed of 6,300 rpm. The product obtained was an unstable, white, free-flowing powder suffering from approximately 5% liquid separation.

### Example 2 (comparative)

To a 250ml glass beaker equipped with an IKA RE166 tooth-disc laboratory high-shear mixer were added 31.1g tap water, 65g betaine and 3.9g CAB-O-SIL TS-720. The contents of the beaker, having an ambient temperature of 22°C, were mixed for 2 minutes at a mixing speed of 2,500 rpm. The product obtained was a stable, white, free-flowing powder. The powder did not show any liquid separation. The aqueous phase contained undissolved crystals of betaine.

### Example 3 (comparative)

The procedure described above in Example 2 was repeated except that 38.0g tap water, 60.0g betaine and 2.0g CAB-O-SIL TS-720 were used. The product obtained, following the mixing of the components, was an unstable cream with 40% liquid separation.

### Example 4 (comparative)

The procedure described in Example 2 was repeated except that 32.0g tap water, 60.0g betaine and 8.0g CAB-O-SIL TS-720 were used. The product obtained after mixing the components was a stable, white, free-flowing, powder which was excessively dusty but which exhibited no liquid separation.

### Example 5

The procedure described in Example 1 above was repeated except that 56.1g tap water, 40.0g betaine and 3.9g CAB-O-SIL TS-720 were used. The product obtained, following the mixing of the components, was a stable, white, free-flowing powder which exhibited no liquid separation.

### Example 6

The procedure described above in Example 2 was repeated except that 36.1g tap water, 60.0g betaine and 3.9g CAB-O-SIL TS-720 were used. Following mixing, the product obtained was a stable, white, free-flowing powder exhibiting no liquid separation.

A comparison of the characteristics of the products obtained in Examples 1 to 6 above is shown in the Table below.

**TABLE**

| **Ex.No.** | **Betaine, %m/m** | **CAB-O-SIL TS-720, %m/m** | **Water, %m/m** | **Density, g/ml** | **Appearance** |
|---|---|---|---|---|---|
| 1 | 30.0 | 3.9 | 66.1 | Approx. 0.60 | Unstable, white, free-flowing powder. Approx. 5% liquid separation. |
| 2 | 65.0 | 3.9 | 31.1 | 0.60 | Stable, white, free-flowing powder. No liquid separation. Some crystals of Betaine. |
| 3 | 60.0 | 2.0 | 38.0 | Not determined | Unstable cream with 40% liquid separation. |
| 4 | 60.0 | 8.0 | 32.0 | 0.37 | Stable, white, free-flowing powder. No liquid separation. Excessively dusty. |
| 5 | 40.0 | 3.9 | 56.1 | 0.50 | Stable, white, free-flowing powder. No liquid separation. |
| 6 | 60.0 | 3.9 | 36.1 | 0.62 | Stable, white, free-flowing powder. No liquid separation. |

### Example 7

A skin-care formulation was prepared as follows.

### Skin-care formulation

| | |
|---|---|
| 76.00% (by weight) | isopropyl myristate (Estol IPM 1512; Uniqema) |
| 6.80% " | lanosterol (Lanosterol BP; Solvay Pharmaceuticals) |
| 2.20% " | phosphatidyl choline (Phospholipon 80G; Phospholipid) |
| 15.00% " | composition according to Example 6 above |

The skin-care formulation above was prepared by first dissolving the lanosterol in the isopropyl myristate at 90°C. After obtaining a clear solution, the solution was cooled to 65°C, followed by dissolution of the phosphatidyl choline while stirring with a planetary mixer. The mixture was stirred until it was fully homogeneous. The temperature of the mixture was then adjusted to 50°C and the composition prepared in accordance with Example 6 was added and the mixture was stirred until it was homogeneous. Subsequently, the product was transferred to another container and allowed to cool during which a gel structure developed. The resultant gel was allowed to age for 24 hours before it was used in the following performance tests.

### Test Panel

A panel of volunteer testers was assembled. The test panel was composed of 10 individuals (4 Caucasian, 3 Mediterranean and 3 Celtic skin types). All 10 members of the test panel were healthy and did not have any skin disorders. The age distribution was:

| | |
|---|---|
| Age 20 - 45 | 5 females, 1 male |
| Age > 45 | 2 females, 2 males |

### Testing Procedure

To determine the efficacy of the betaine composition of Example 6, determination of the TEWL values (TransEpidermal Water Loss) and corneometer readings were performed. The TEWL values give information on the water flux of water from the organism to the outer world (or vice versa). Corneometer experiments give information on the amount of organically bound water in the subcutaneous tissue.

The skin can be considered as a capacitor. The dielectric constant of the skin as a capacitor is dependent on the amount of organically bound water and is thus representative for the degree of hydration. Thus, the dielectric constants of water and "dry" organic tissue material are different, and consequently the water content of the subcutaneous tissue can be directly measured by measurement of the dielectric constant.

Corneometer experiments were performed using the Corneometer CM 825 (Courage & Khazaka). TEWL values were determined using the Tewameter TM 210 (Courage & Khazaka). The environmental temperature was very high due to seasonal constraints (27.4°±0.5°C); the relative humidity was 84±5%. These conditions were assumed not to represent average conditions.

Before the test substances were applied on the skin on the inner side of the right and left forearm, the test locations were wetted with water using a regular spray bottle. After 15 seconds, the excess water was removed using a cotton pad, to such an extent that visual water was completely removed. The skin was allowed to condition during 1 minute. Subsequently, the test formulation was applied on the inner right forearm.

A skin area of 16 cm² was reserved for the measurements. An amount of 1.5g was applied to the skin and enabled to be absorbed into the skin during 2 minutes, without application of shear. The excess material present after 2 minutes was removed using a cotton pad. The measurements were done after 5 minutes, 30 minutes, 1 hour and 2 hours after application. Each volunteer was subjected to at least 10 experiments on different parts of the skin of the under arm where the product was applied; for a particular time experiment; the results were averaged. Reference measurements were done on the remaining part of the skin inner forearm; also here 15 experiments were done; the results were averaged.

The obtained results were averaged and the standard deviation was determined. Readings outside the range -2σ < Φ <2σ were rejected.

### Results

From the corneometer experiments 2 out of 10 experiments were rejected; the TEWL experiments did not result in rejections.

### Corneometer Experiments

The corneometer experiments showed a significantly increased capacity reading as a function of time: 24±5% after 30 minutes. After 1 hour the performance was 21±4%, while after 2 hours the performance was 14±2%. Using the Marquardt procedure for non-linear parameter optimisation, it was calculated that a 20% decaying increase was reached after 5.5 hours. A 10% decaying increase was reached after 9 hours, and this is considered as significant.

### Transepidermal Water Loss Experiments

The TEWL experiments showed a slightly reduced TEWL. After 2 hours, the transepidermal water loss was less than 2% versus standard. This indicates that the flux of water from the skin to the outer world is not influenced by the application of the test formulation. This was an appreciated finding.

### Conclusions

Despite the poor environmental measurement conditions, the results are excellent. It was demonstrated that application of the test formulation results in a marked positive effect. It was also concluded that the corneometer readings do not go hand-in-hand with the TEWL readings.

### Example 8

### Test Panel

The test panel was composed of 11 females (6 Caucasian, 3 Mediterranean and 2 Celtic skin types) and 13 males (6 Caucasian, 5 Mediterranean and 2 Celtic skin types).

All 24 volunteers were healthy and without skin disorders. The age distribution ranged from 25-67, distributed as follows:

| | |
|---|---|
| Age 20 - 30 | 3 females, 4 males |
| Age 30 - 40 | 6 females, 7 males |
| Age 40 - 50 | 1 female, 1 male |
| Age > 50 | 1 female, 1 male |

A cosmetic formulation was prepared, for testing, as follows. Firstly, a matrix composition containing the following ingredients was prepared:

| | |
|---|---|
| 62.30% (by weight) | isostearyl isostearate (Prisorine 2039; Uniqema) |
| 10.00% " | Jojoba oil (Jojoba Oil Golden; Gustav Heess) |
| 7.00% " | dextrin palmitate (Dextrin Palmitate 49282; Nikko Chemicals) |
| 5.40% " | isopropyl myristate (Estol 152; Uniqema) |
| 3.50% " | lanosterol (Lanosterol BP; Solvay Pharmaceuticals) |
| 1.60% " | phosphatidyl choline (Phospholipon 85G; Phospholipid) |
| 0.20% " | antioxidant (Oxynex K Liquid; Merck) |

The matrix composition was prepared by first dissolving phosphatidyl choline at 45°C in 20% of the isostearyl isostearate, in the presence of an antioxidant. This was done separately to avoid excessive oxidation of the unsaturated phosphatidyl choline due to exposure to high temperature. The remaining ingredients were combined by heating the mixture to 90°C while stirring with a planetary mixer. A homogenous solution was obtained, that was cooled to 60°C, at which temperature the gel was not yet formed. The solution of phosphatidyl choline (@ 45°C) was added to the solution of the remaining ingredients, and stirred until fully homogeneous. At 50°C the obtained gel was transferred to a suitable container. The gel structure developed in the container to a fully transparent gel, with a high degree of thixotropy and shear thinning.

The viscosity, measured at 20°C using a Brookfield DV-II+ Programmable Viscosimeter using a T-bar spindle @ 5 rpm consistently decays as a function of time; measurement time 10 minutes. The starting viscosity was 42.000 cPs, decaying to 13.000 cPs after 10 minutes. This process was fully reversible. The yield stress value, determined after 30 seconds, was 115 Pa, sufficient to prepare a stable dispersion.

The cosmetic formulation was prepared by mixing, at 35°C, the betaine composition prepared in Example 6 above (20% by weight of the formulation) and the above-prepared matrix composition (80% by weight of the formulation), in a planetary mixer. A homogeneous, non-transparent dispersion was obtained. The dispersion was allowed to age for 24 hours before use in the testing procedure.

Both the matrix composition and the cosmetic formulation (containing the betaine-containing composition of Example 6) were tested for free water using powdered, anhydrous cobalt nitrate. The tests were carried out 24 hours after and, again, 5 days after preparation. Anhydrous cobalt nitrate, when exposed to a composition containing at least 0.02% free water produces a blood red colouration in the composition. It was found that neither the matrix composition nor the cosmetic formulation produced any colouration (after 24 hours or after 5 days from preparation) when contacted with anhydrous cobalt nitrate, thus indicating that neither contained any detectable free water. It was concluded, therefore, that the formulation was stable.

### Testing Procedure

To determine the efficacy of the formulation containing the betaine encapsulated composition of Example 6, combined determination of the TEWL values (TransEpidermal Water Loss) and corneometer readings were performed. The TEWL values give information of the water flux from the organism to the outer world (or vice versa). Corneometer experiments give information on the amount of organically bound water in the subcutaneous tissue.

As described in Example 7 above, for the corneometer experiments, the Corneometer CM 825 (Courage & Khazaka) was used. For the TEWL value determination, the Tewameter TM 210 (Courage & Khazaka) was used. The environmental temperature was conditioned at 22±0.5°C; the relative humidity was set at 65±3%.

Before the test substances were applied on the skin of the inner side of the right and the left forearm, the test locations were wetted with water using a regular spray bottle. After 15 seconds, the excess water was removed using a cotton pad, to such an extent that visual water was completely removed. The skin was allowed to condition during 1 minute. Subsequently, the test cosmetic formulation was applied on the inner right forearm and the matrix composition was applied on the left inner forearm.

A skin area of 16 cm² was reserved for the measurements. An amount of 1.5g was applied to the skin and enabled to be absorbed into the skin during 2 minutes, without application of shear. The excess material present after 2 minutes was removed using a cotton pad. The measurements were done after 5 minutes (set 1), 30 minutes (set 2), 1 hour (set 3) and 4 hours (set 4) after application. Each volunteer was subjected to 15 experiments on different parts of the skin where the products were applied; for a particular time experiment; the results were averaged. Reference measurements were done on the remaining part of the skin inner forearm; also here 15 experiments were done; the results were averaged.

The obtained results were averaged and the standard deviation was determined. Readings outside the range -2σ < Φ <2σ were rejected.

### Results

### Placebo Experiments (using the matrix composition not containing the betaine composition of Example 6)

From the set of 24 corneometer experiments, set 1 gave 2 rejections, set 2 gave 2 rejections, set 3 gave 1 rejection and set 4 gave 4 rejections. The TEWL experiments gave no rejections.

The placebo corneometer experiments versus the reference experiments showed only a slightly increased capacity and thus only a very slightly increased degree of hydration (2%). This result is attributed to the application of the placebo to the skin, and is considered not significant.

The placebo TEWL experiments versus the reference experiments showed no difference change in the TEWL values. It was concluded that the application of the placebo has no impact on the performance of the skin relative to the moisture balance.

### Experiments using the cosmetic formulation (containing the betaine composition of Example 6)

From the set of 24 corneometer experiments, set 1 gave 3 rejections, set 2 gave 4 rejections, set 3 gave 2 rejections and set 4 gave 5 rejections. From the set of 24 TEWL experiments, set 1 gave 1 rejection, set 2 gave 1 rejection, set 3 gave 2 rejections and set 4 gave 3 rejections.

### Corneometer Experiments

The corneometer experiments using the cosmetic formulation versus the reference matrix composition showed a highly increased capacity reading as a function of time (see Table 2). The data are expressed as a relative measurement.

**Table 2: Corneometer readings as a function of time**

| **Time (minutes)** | **Reading** |
|---|---|
| 0 | 39 |
| 5 | 42 |
| 30 | 64 |
| 60 | 60 |
| 240 | 53 |

Using the Marquardt procedure for non-linear parameter optimisation, it was calculated that a 20% decaying increase was reached after 7.5 hours. A 10% delaying increase was reached after 12 hours, and this is considered as highly significant.

### Transepidermal Water Loss Experiments

The TEWL experiments using the cosmetic formulation versus the reference matrix composition experiments showed a relatively modest suppressed water loss. The application of the matrix composition did not result in a decreased TEWL value. After application of the cosmetic formulation, a modest decrease of the TEWL value was observed (see Table 3).

**Table 3: TEWL readings as a function of time**

| **Time (minutes)** | **TEWL (%)** |
|---|---|
| 0 | 0.0 |
| 5 | - 1.2 |
| 30 | - 19.6 |
| 60 | - 11.8 |
| 240 | - 6.0 |

Using the Marquardt procedure for non-linear parameter optimisation, it was calculated that a 20% decrease was reached after 4.2 hours. A 10% decrease was reached after 6.1 hours, and also this result is considered as significant.

### Sensorial Appreciation

The panellists were generally of the opinion that the matrix composition softens the skin remarkably. This was expected as softening of the (dead) stratum corneum easily absorbs a significant amount of lipids. Frequently, this is correlated with a high degree of moisturisation. It was demonstrated that this is not the case.

Application of the matrix composition and the cosmetic formulation did not show a difference in perception; the timescale was too short to enable the panellists to distinguish between both products from a sensorial point of view.

However, three panellists voluntarily continued to use both products during one week, using the "half body" approach, not being aware of the identity of the products applied. Their remarks were that after 3-4 days, the long term effects of the cosmetic formulation were clearly noticeable and superior compared to the matrix composition not containing the betaine composition of Example 6. All three panellists were unanimous; no quantitative data have been made available.

### Conclusions

It was demonstrated that application of the formulation containing the betaine composition of Example 6 results in a marked positive effect relative to placebo (matrix composition). It was also concluded that the corneometer readings do not go hand-in-hand with the TEWL readings. That is not surprising as transepidermal water loss is mainly a translation of the cooling mechanism of the organism while moisture retention on a cellular level (made visible using corneometer experiments) is part of the metabolic pathways that take place.

It was concluded that formulation containing the betaine composition has an outspoken and positive effect on the moisture housekeeping on a cellular level.

## Claims

1. A particulate composition comprising components (A) finely particulate hydrophobic silicone-treated silica having a surface area of from 80 to 300 m²/g, (B) trimethylglycine, and (C) water, wherein the content of component (A) is greater than 2% and less than 8% by weight based on the total weight of components (A), (B) and (C) and wherein the content of component (B) is greater than 30% and less than 65% by weight based on the total weight of components (A), (B) and (C), said composition being in the form of particles of an aqueous solution of component (B) the surfaces of which particles are coated with a coating of component (A).

2. A particulate composition according to claim 1, wherein the content of component (A) is in the range of from 3 to 5% by weight based on the total weight of the components (A), (B) and (C).

3. A particulate composition according to either claim 1 or claim 2, wherein the content of component (B) is in the range of from 40 to 62% by weight based on the total weight of the components (A), (B) and (C).

4. A particulate composition according to claim 3, wherein the content of component (B) is about 60% by weight based on the total weight of the components (A), (B) and (C).

5. A particulate composition according to claim 4 comprising 3.9% by weight of component (A), 60% by weight of component (B) and 36.1 % by weight of component (C), all % by weight based on the total weight of components (A), (B) and (C).

6. A method of making a composition according to claim 1 comprising the steps of subjecting a mixture of component (A), finely particulate hydrophobic silicone-treated silica having a surface area of from 80 to 300 m²/g, and an aqueous solution obtained by dissolving component (B), trimethylglycine, in component (C), water, to shear in a high-shear mixer operated at a mixing speed in the range of 2000 to 7000 rpm for 1 to 10 minutes, wherein the amount of (A) is greater than 2% and less than 8% by weight based on the total weight of (A), (B) and (C) and the amount of (B) is greater than 30% and less than 65% by weight based on the total weight of (A), (B) and (C).

7. The use of a composition according to any one of claims 1 to 5 as a moisturiser in a liquid formulation for topical application to the skin.

8. A formulation for application to the skin comprising a particulate composition according to any one of claims 1 to 5 and a dermatogically-acceptable or cosmetically-acceptable carrier, wherein the particulate composition is present in an amount such that the betaine content of the formulation is within the range of from 0.05 to 50% by weight.

9. A formulation according to claim 8, wherein the particulate composition is present in an amount such that the betaine content of the formulation is within the range of from 0.5 to 10% by weight.

10. A formulation according to claim 9, wherein the particulate composition is present in an amount such that the betaine content of the formulation is within the range of from 2 to 5% by weight.

11. A formulation according to any one of claims 8 to 10 which is a dermatological formulation or a cosmetic formulation.

12. A cosmetic formulation according to claim 11 which comprises one or more ingredients selected from emollients, humectants, fragrance additives, phospholipids, vitamins, stabilizers and antioxidants.
